# EUROPEAN PATENT SPECIFICATION

(11) **EP 1 277 051 B1**
(45) Date of publication and mention of the grant of the patent: **23.08.2006**
(21) Application number: 01914241.3
(22) Date of filing: 19.02.2001
(51) Int. Cl.: G01N 33/24, G01V 9/00, E21B 47/10

(54) **RESERVOIR MONITORING**
ÜBERWACHUNG EINES RESERVOIRS
SYSTEME DE CONTROLE D'UN RESERVOIR

(30) Priority: 26.04.2000 NO 20002137; 29.11.2000 US 725042
(43) Date of publication of application: 22.01.2003
(73) Proprietor: ResMan AS, 7491 Trondheim (NO)
(72) Inventor: TAYEBI, Davoud, N-7050 Trondheim (NO); KILAAS, Lars, N-7014 Trondheim (NO); LUND, Are, N-7048 Trondheim (NO); KVERNHEIM, Arne, Lund, N-1450 Nesoddtangen (NO); ERIKSEN, Odd, Ivar, N-0379 Oslo (NO); SVEEN, Jostein, N-7052 Trondheim (NO); LILE, Ole, Bernt, N-7021 Trondheim (NO); RAMSTAD, Marit, Valeur, N-7049 Trondheim (NO); SAASTAD, Ole, Widar, N-0468 Oslo (NO)
(74) Representative: Haley, Stephen
(86) International application number: PCT/NO2001/000059
(87) International publication number: WO 2001/081914

(56) References cited:
- US-A- 3 991 827
- US-A- 4 008 763
- US-A- 4 055 399
- US-A- 4 555 489
- US-A- 5 077 471
- US-A- 5 212 093
- US-A- 5 892 147

## Description

### FIELD OF THE INVENTION

The present application relates to a method for monitoring the hydrocarbon and water production from different production zones/sections in a hydrocarbon reservoir and detection of different phenomena such as e.g. local variations in pH, salinity, hydrocarbon composition, temperature, pressure, microorganisms, and the difference/ratio between production of formation and/or injection water from various zones/sections in wells in a hydrocarbon reservoir. The method enables both short time and long time monitoring and detection of the above mentioned phenomena. Uses of the invention are also disclosed.

### BACKGROUND OF THE INVENTION

A geological porous and permeable formation in the ground containing hydrocarbons is called a hydrocarbon reservoir. A hydrocarbon reservoir rock type consists of two elements, the matrix, which are the solid rock fragments, and the pore volume in between the rock fragments. The pore volume constitutes the porosity, ϕ, which is the fraction of pore volumes relative to the total volume that is set equal to 1.0. For example, ϕ=0.3 means that 30% of the total volume is pore volume and the rest of the volume (70%) is the rock matrix.

In a hydrocarbon reservoir the pore volume is generally filled with water, oil and/or gas. Due to density differences, the hydrocarbons will accumulate in the upper part of the reservoir. Water will occupy the pore spaces below the hydrocarbon zone in the reservoir. Between the hydrocarbon zone and the water zone below (the aquifer), there is a transition zone where the hydrocarbon saturation drops from close to 100% down to 0%, representing the Oil-Water-Contact, OWC.

During the production of hydrocarbons, water is normally displacing the hydrocarbon and the OWC level will rise in the reservoir. After some period of production, the water fraction of the produced fluids will increase. Generally, the fraction of water in the total volume of produced fluids increases steadily. Finally, the water fraction will be too high and the production becomes non-profitable. Often the production may come from different layers or zones of the formation cut by the well. The individual zones may have different permeability and will normally behave differently during production. It is generally an advantage to know this behavior. The gathering and analyses of information from the reservoirs during the production is called Reservoir Monitoring. The typical types of collected data are chemical compostion, temperature, pressure, electrical resistivity, and water saturation in the reservoir close to the production well. Other types of information may be seismic maps of the whole reservoir at different stages of the hydrocarbon production.

When hydrocarbons are produced from a porous and permeable geological formation in the ground, a reservoir, the flow of hydrocarbons and water can come from different zones or layers of the reservoir. To monitor and analyze the production of different fluids, it is often important to get information about the type of fluids and the amount of the various components that flow from each zone.

A known method to examine the local flow properties in an oil and/or gas producing well is to lower a logging tool into the well. This is described e.g. in US 4861986, US 5723781 and US 5881807.

To examine the local production the logging equipment lowered into the well measures the amount of oil and/or gas flowing in the well at different places along the well. Using this technique it is possible to calculate the amount of oil flowing into any region of the well. Among the disadvantages of this technique is that the production of oil and/or gas has to be wholly or partially stopped during the logging. This is a major disadvantage since one wishes to measure the local amount of oil and/or gas flowing in the well during the logging. Using this technique, it is often difficult to distinguish between the amount of oil and water in a mix flow. For a long horizontal borehole (4-6 km) it is very difficult or almost impossible to use the technique since special leading equipment is required to insert the logging probe into the horizontal wells. Furthermore, putting logging equipment into a well reduces the cross-sectional area of the producing well, resulting in larger pressure drop. The method is also very expensive.

Another known technique in reservoir study is the application of traceable materials. Tracers are used to determine size and shape of reservoirs. US 4420565 and US 4264329 describe methods for following fluid flow in underground reservoirs using tracer materials. In US 4420565 the depth of recovery is determined by injecting a solution containing a small amount of one or more watersoluble tracer compounds into the formation at a predetermined depth from the injection system and recovering it in the production system. The tracer compounds are then identified using gas chromatography and a flame ionization detector. US 4264329 uses metal chelates as tracers, and liquid chromatography and fluorescence spectroscopy to detect the metal chelates in the produced fluid.

US 3991827 and US 4008763 describe a method for determination of solid particle leakage into the wellbore from different solid particle packs placed along the well using tracer particles. High concentration (50-70% by weight) of tracer particles are packed in a plurality of solid particle packs which are placed in a well to prevent solid production from the formation into the wellbore. The tracer particles used are unique for each particular pack, for example particles with different colours. If any of the solid packs is leaking during the production, the tracer particles from that pack or packs will follow the produced fluid that is analyzed at the surface to determine which pack is leaking solid particles. Using this technique, subsequent workover of the well can be limited to the pack or packs where the leaking packs are identified, rather than being directed to the whole well.

The technique described in US 3991827 and US 4008763 is a method for short time detection of leaking packages. Very high concentrations of tracer particles (50-70% by weight) are being used, where tracer particles are mixed with the packing particles and placed in packs around the well. There is no chemical bond between tracer particles and packing materials. The tracers will follow the flow regardless of the fluid type. The packs leaking particles will lose a large amount of tracers in a short time and therefore are not suitable for long time monitoring. In addition, migration of large amount of solid particles such as sand and fines can wear out pipes, valves and other equipment. On the other hand, the tracers will be captured in the packs that do not leak, and therefore no information will be available from those regions. The method as it is described is useable only for examining the solid particle leakage.

US 5892147 describes a procedure for determination of oil and/or gas inflow from a reservoir into a wellbore. Different radioactive isotopes are used as tracers placed in different zones along the well. The radioactive isotopes are fixed either on the outside of the transport pipe or inserted into the formation through the well casing using perforation guns. Based on the amount of the measured tracers the amount of oil and/or gas flowing into the well at each zone is calculated.

In both cases described in US 5892147, the radioactive isotopes are withdrawn by the fluid as a result of the fluid flow, where the fluid could be oil, water or gas. Based on this technique it is therefore difficult to judge which zone producing what. Fixing the radioactive isotapes on a surface of a transport pipe is limited only to those wells having a transport pipe with the arrangement required for this technique. In the other alternative the radioactive isotape particles are inserted into the formation as a load to an explosive charge which is shot through the well casing. The tracers are washed out with the first patches of fluid flow through the perforation holes since they most likely lie free on the interior surface of the perforation holes without any chemical bond to the formation. This method therefore gives some information about the inital phase of the production in each zone. In addition, the application of radioactive isotopes as tracers is expensive and requires special handling due to health, environmental and security reasons.

The present invention has been conceived to solve or at least alleviate the problems of the prior art as described above. According to the present invention there is provided a method for monitoring hydrocarbon and water production from a hydrocarbon reservoir or injection well and detection of phenomena including local variations in pH, salinity, hydrocarbon composition, temperature, pressure, microorganisms, and a difference between production of formation and injection water, the method comprising the steps of:
dividing regions around wells in the reservoir into a number of zones, and
characterised by the steps of:
chemically immobilizing specific chemically intelligent tracers with unique characteristics for each zone into a well completion, a filter, a casing or construction surrounding the well in each zone, releasing the tracers as a function of a specific event, wherein said tracers are immobilized by means of at least one polymer capable of adhering to the formation, and wherein the tracers being covalently bonded or bonded by ionic interactions to said polymer, and
detecting the tracers downstream to monitor said zones

The regions around wells in a reservoir are divided into a number of zones/sections, and specific tracers with unique characteristics for each zone/section are placed as integrated parts of the well completion. The tracers are chemically immobilized or integrated into the formation or in/on the constructions/filters around the wells. The tracers and/or tracer carriers are chemically intelligent and released as a function of specific events. Tracers are detected downstream somewhere along the production line or on the surface, providing information about the various zones/sections in the reservoir.

Among the many advantages of the present invention is the determination of local and detailed information about the above mentioned phenomena, using a fluid intelligent tracer technique together with a relatively simple data detection system which requires no equipment down-hole during production. Therefore the well hole remains unchanged resulting in lower pressure drop. Since there is no equipment down-hole, there is no need for equipment maintenance either. The method gives both short time and long time information which can be used for example for early warning, start phase detection, or long time monitoring, etc. The method uses no radioactive isotopes, or any material harmful to the environment.

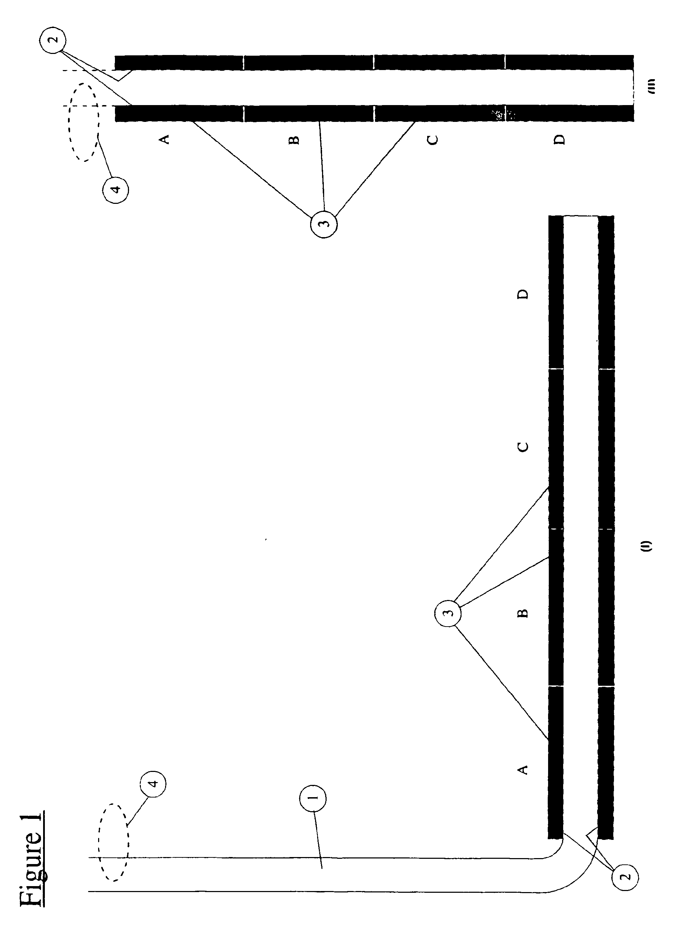

The present invention provides a fluid intelligent and reliable tracer technique for short time and long time monitoring of different production zones/sections in oil and gas production wells and also for detection or early warning of phenomena such as water breakthrough, local variations in pH, salinity, hydrocarbon composition, temperature, pressure and microorganisms. The method enables also measurement of the difference/ratio between production of formation and/or injection water from various zones/sections in a hydrocarbon reservoir. The main advantage of this invention is that it makes it possible to collect the local detailed information from each individual zone of the reservoir, and production and/or injection wells using a chemical fluid intelligent tracer technique. The well or wells in a reservoir are divided into a certain number of separate zones/sections equipped with proper tracers of unique characteristics for each zone/section as illustrated in Figure 1. The tracers are placed as integrated parts of the well completion chemically immobilized into the formation and/or in/on the constructions or filters around the wells. The tracers can be immobilized on the filter or the casing around the well in each zone/section. The tracers and/or tracer carriers are chemically intelligent and released as a function of specific events. Tracers are detected downstream somewhere along the production line or on the surface/platform. The collected information can be used to study the local flow conditions in each zone/section and to describe the reservoir behavior during production. Such information includes for example production rate, location and the type of produced fluids, e.g. how much oil, water or gas is produced and from where in the reservoir they come. The method can be used to study the reservoir dynamics behavior in the neighborhood of the production and injection wells. The method can be used to detect the phenomena such as water breakthrough, variations in pH, salinity, hydrocarbon composition, temperature, pressure and microorganisms in each well section/zone. Using intelligent tracers it is also possible to distinguish between produced injection water and formation water from each zone/section. The technique can be also used as a local alarm- or early warning system for both quantitative and qualitative detection of the appearance of phenomena such as water breakthrough, variations in pH, salinity, hydrocarbon composition, temperature, pressure, and microorganisms. The invention can in principle also be used for other production processes and fluid transport facilities.

### BRIEF DESCRIPTION OF DRAWINGS

The above and further advantages may be more fully understood by referring to the following description and accompanying drawings of which:
Figure 1 is a schematic illustration of the horizontal part (I) and/or the vertical part (II) of a well in a reservoir 2 divided into a certain number of zones/sections 3, e.g. A-D according to the invention equipped with specific tracers with unique characteristics for each zone/section.

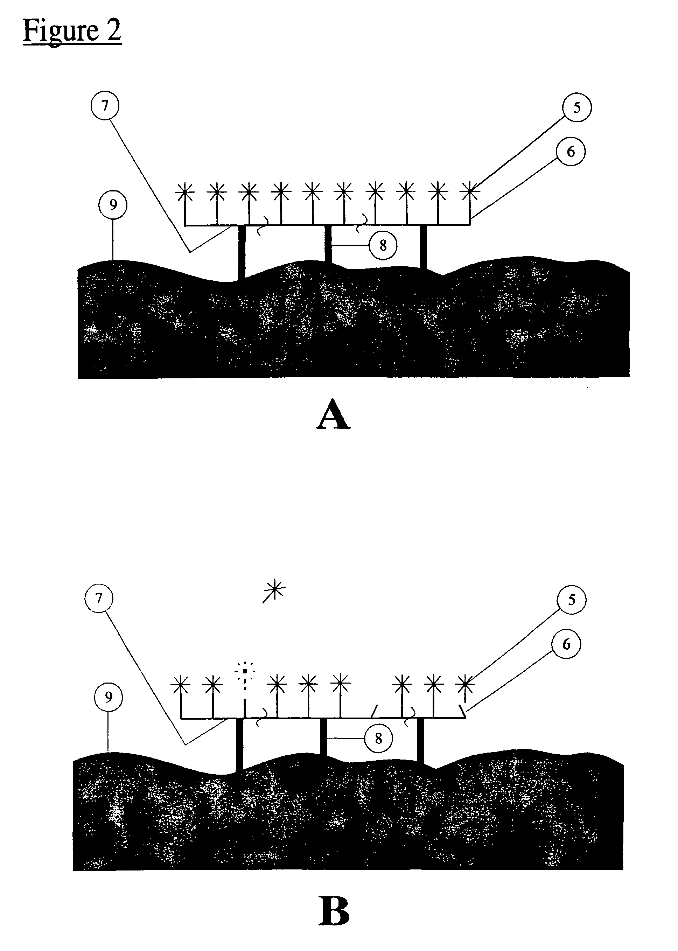

Figure 2(A) illustrates schematically a number of tracers 5 chemically immobilized 6-8 on e.g. the pore wall of a rock matrix or a polymer matrix, the surface of a sand particle, a polymer particle, an inorganic particle, or on any other compact or porous surface or matrix 9. Figure 2(B) schematically illustrates a tracer 5 being released from the formation 9 as a result of a chemical reaction leading to the breakage of bond 6 between the tracer 5 and binding molecule 7.

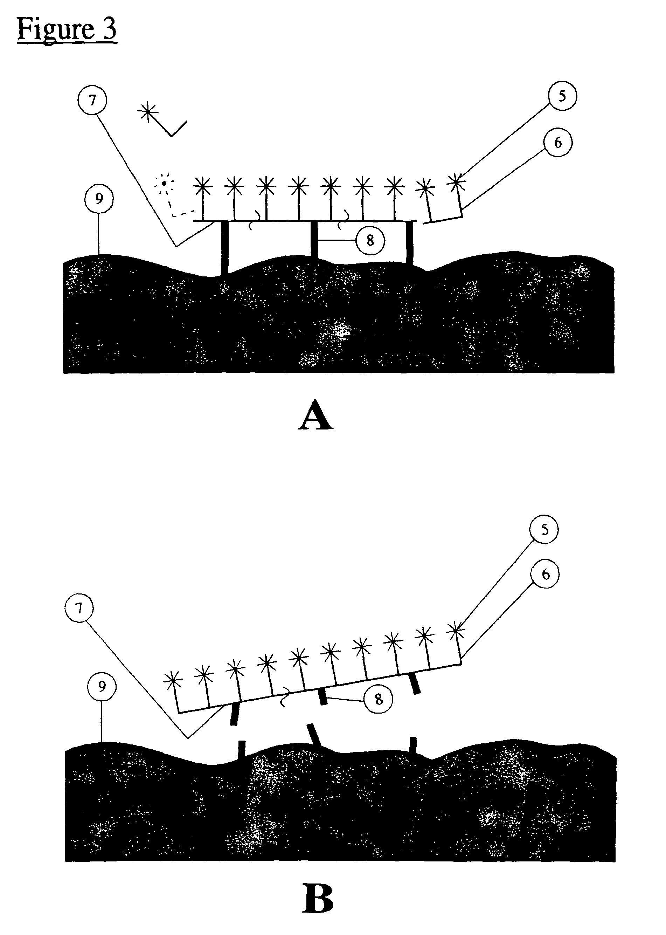

Figure 3(A) schematically illustrates one or several tracers 5 being released from the formation 9 as a result of a chemical reaction leading to the breakage of a bond in the binding molecule 7 for the tracers 5. Figure 3(B) schematically illustrates one or several tracers 5 being released from the formation 9 as a result of a chemical reaction leading to the breakage of bond 8 between the binding molecule 7 and the surface 9.

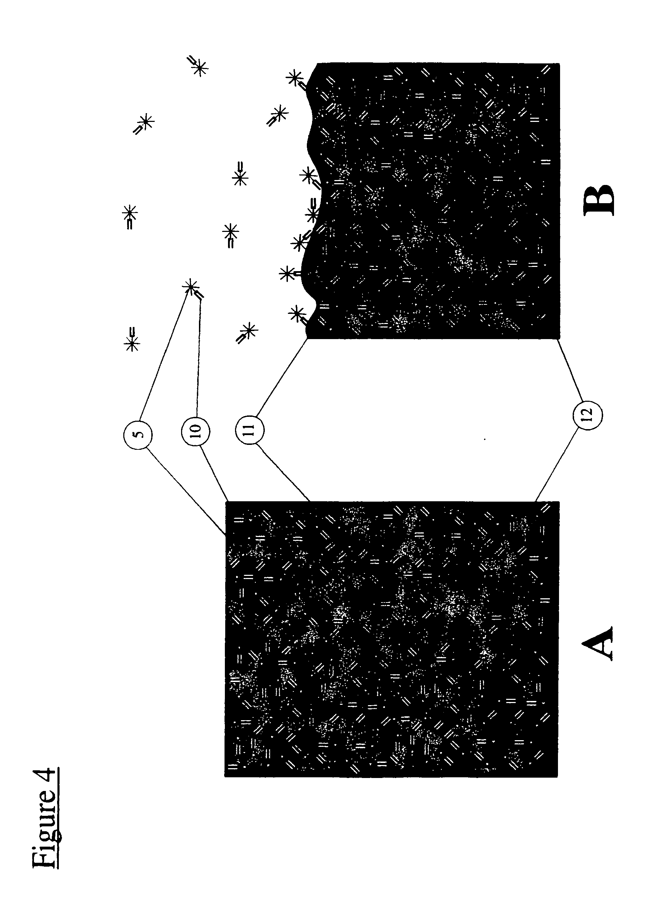

Figure 4(A) schematically illustrates a number of tracers 5 being packed/immobilized into a small package 12, e.g. a chemically intelligent gel or a polymer package 11. The tracers 5 are chemically bonded to the packing material 11 by a chemical bond (or other types of weak forces) 10. The package 12 may have any shape or size e.g. such as small capsules, tablets, polymer particles or polymer matrixes. Figure 4(B) illustrates schematically the gradually release of tracers 5 from the package 12 as a result of a chemical reaction leading to degradation or decomposition of the packing material 11 and release of the tracer 5.

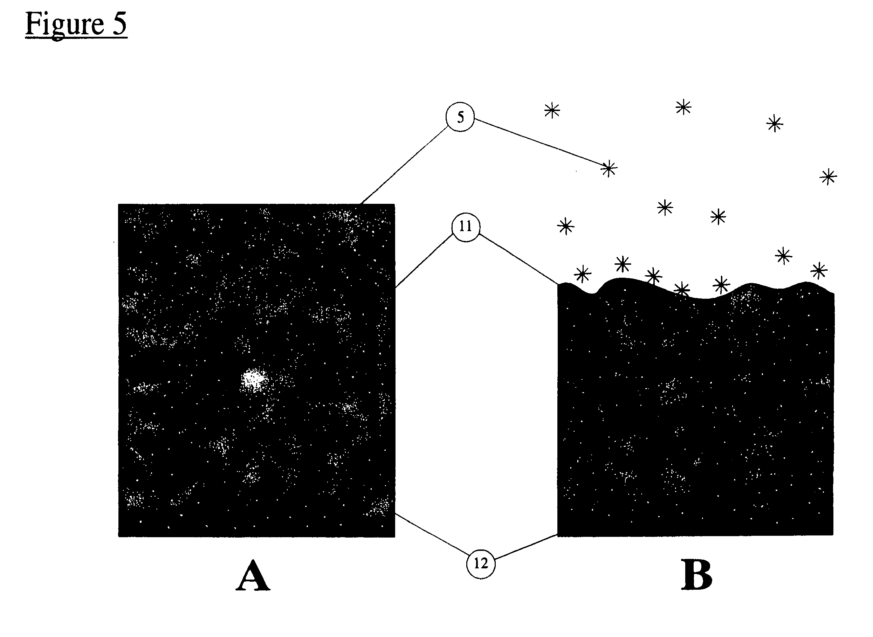

Figure 5(A) schematically illustrates a number of tracers 5 being packed/immobilized into a small package 12, e.g. a chemically intelligent gel or a polymer package 11. The package 12 may have any shape or size e.g. such as small capsules, tablets, polymer particles or polymer matrixes. Figure 5(B) illustrates schematically the gradually release of tracers 5 from the package 12 as a result of a chemical reaction leading to degradation or dissolution of the packing material 11.

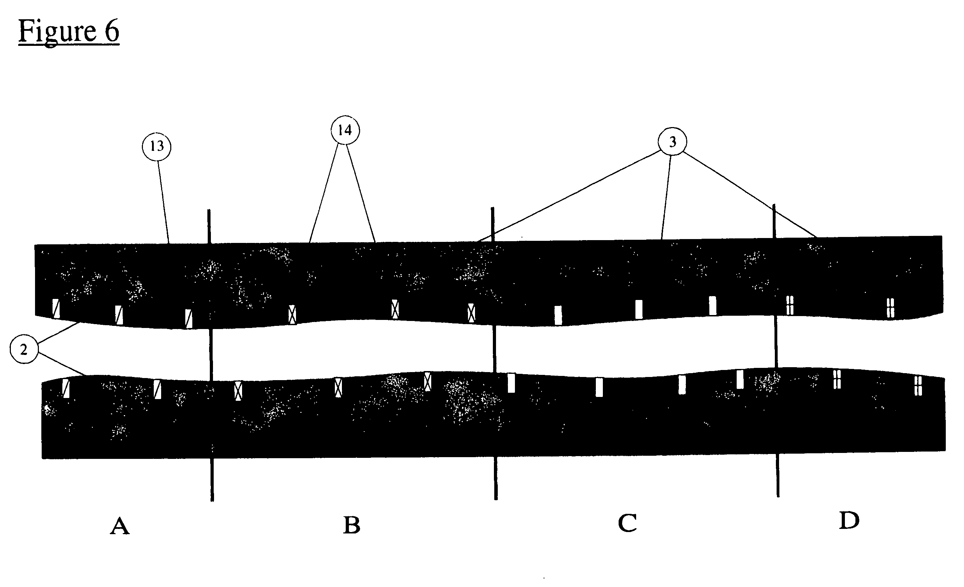

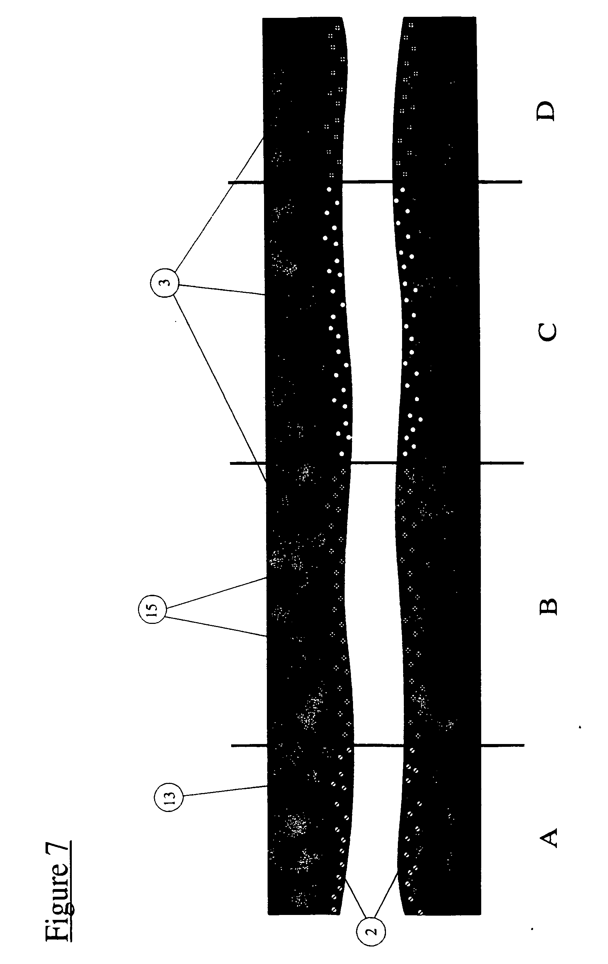

Figures 6 and 7 schematically illustrate different ways of locating tracer packages in various zones/sections along the well in a reservoir formation. The tracers are e.g. placed along the well in a certain pattern or injected into the formation, e.g. as swellable porous or solid polymer matrixes.

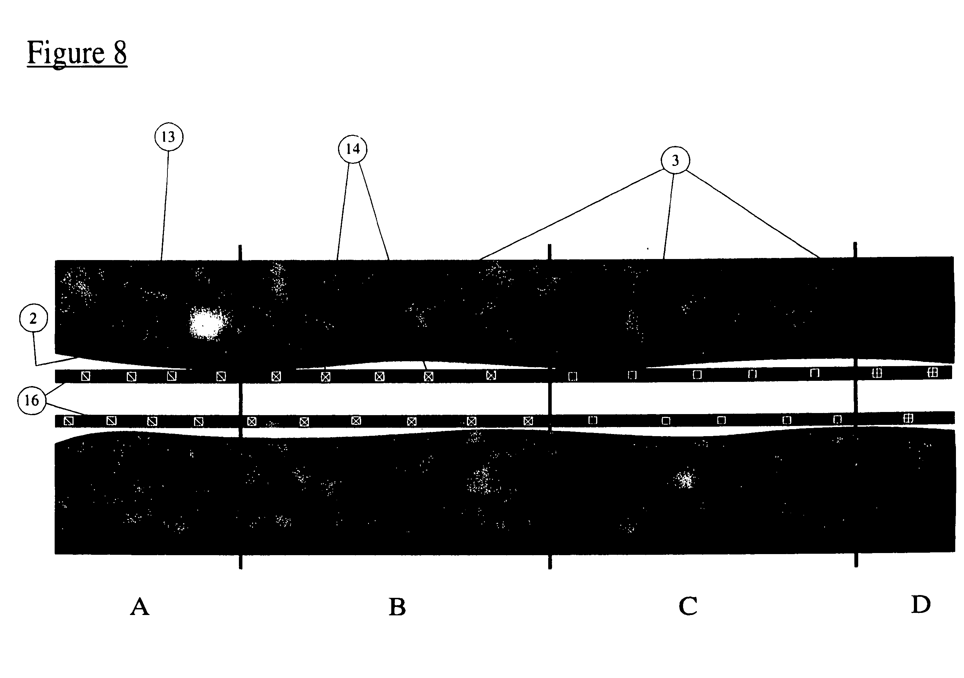

Figure 8 schematically illustrates locating tracer packages in various zones/sections along the well in the constructions or filters around the well. The tracers are e.g. placed along the well in certain patterns, injected into the constructions or filters around the well, mixed with the material in the filter content, or fixed on the constructions or filters around the well.
   The release process for tracers in all the above mentioned methods could be as those illusuated in Figures 2-5 or any of their combinations.

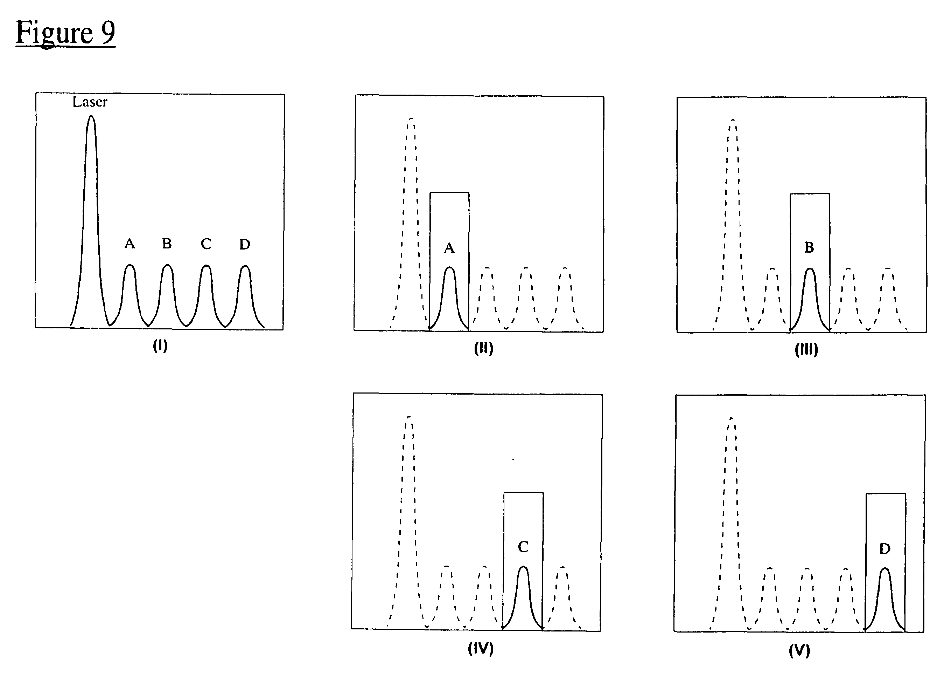

   Figure 9 is a schematic illustration of the method for selection of the desired light spectra for detection of individual tracer particles with different emission wavelength using optical filters. (I): Hole spectra of the reflected and emitted lights from the carrying fluid and tracer particles respectively. (II), (III), (IV) and (V): Blockage of the unwanted light spectra and transmission of the desired light spectra using proper optical filters.

### DETAILED DESCRIPTION

A series of various traceable materials such as tracer particles, biologically coded materials, design chemicals or fluids can be placed in different known locations, zones or sections in a reservoir along a well or through the injection wells. It is also possible to use specific reservoir microorganisms or rare minerals as tracers. The tracer materials will be chemically immobilized in the reservoir formation along the well,

In cases where there is a filter or other constructions around the well, the tracers can be chemically immobilized or chemically bonded in or on the construction along the well in separate zones/regions. The tracers are e.g. placed along the well with certain patterns, immobilized on the filter, the casing or constructions surrounding the well in each zone/section. The immobilization of tracers on these constructions can for instance be performed before the insertion of the filter or the casing into the well. In this case one may use different tracers or tracer combinations for each part of the fitter or the casing dividing the well into various sections/zones. The tracers will be released gradually as a result of the production of the individual fluids in each specific zone or be triggered/released by specific events like water breakthrough, enzymes or by other mechanisms. The tracer release mechanism could be a degradation process of the matrix holding the tracers by breakage of a bond/bonds between tracers and the reservoir formation or packing material, Figure 3- The released tracers will follow the produced fluids out of the well and can be detected somewhere along the production line downstream. Since the locations of different tracers in the reservoir formation are known in advance, the behavior and the dynamics of the individual production zones (and the event releasing the tracers) along the well can be monitored by detecting the type of tracers coming from different sections.

The tracers are made "chemically fluid intelligent" such that they will be released as a response of specific events, e.g. they respond to an oil flow (oil-active) but show no response to a water flow (water-resistant). Another group of chemical compounds can be placed in the same region, which release tracers in water flow (water-active), but show no response to an oil flow (oil-resistant). This means that when there is no or very little water production, there will be no release of water-active tracers, and vice versa.

Tracers that are released in the gas-phase of a multiphase system are also possible to construct and monitor. Intelligent chemicals or materials sensitive to specific events such as water breakthrough, local variations in pH, salinity, hydrocarbon composition, temperature, pressure, microorganisms, etc. which release specific tracers can also be placed in the same region. These tracers can be used both as an alarm system (early warning) and for the local study of the specific phenomena over time. Specific chemicals can be placed latent in the formation along the well in the reservoir being unique for each well zone/section. These compounds can be activated to release tracers using specific triggers for example through the injection system. Using this technique as release mechanism combined with the injection water will allow the local detection of for example water breakthrough of the injected water. In such a system produced formation water and/or injected water will be detectable by the release of water-active tracers (compounds sensitive to water which release tracers), but the difference/ratio between injection or formation water will be detectable using the above mentioned latently stored tracers.

Two or more time dependent tracer release processes (long time release and short time release process) can be designed using different chemical compounds. In a short time release process large amounts of specific tracers are released in short time, while in the long time release process, small amounts of another group of tracers are released gradually over long time. One or several tracer groups with different release processes and characteristics can be placed in the same region/zone. The purpose of these processes could be different, for example for early warning, for start phase detection, or long time monitoring, etc. Another type of tracer would be the sudden release type. In this case a relative small amount of tracer can be localized in the well to detect some later event that could occur in that zone (type of fluid, pH, salinity changes, temperature, pressure changes etc.).

The tracers can be e.g. fluorescent, phosphorescent, magnetic particles or fluids, easily detected compounds by chromatographic methods, biologically coded materials or rare minerals which are not usually found in the produced fluids from the concerned reservoir. Tracers responding to acoustic signals may also be possible to construct. The tracers can be injected in the reservoir formation in the neighborhood of the well and fixed in place (immobilizedllinked) chemically

Another possibility is to put a compact amount of tracer materials fixed in a fluid intelligent chemical compound in the reservoir formation as a swell-able porous or solid polymer matrixes or particles. In cases where there is a filter or other constructions around the well, the various tracers can also be chemically immobilized in/on the construction along the well in separate zones/regions. The tracers may also be injected into the reservoir through injection wells. The tracers will be released either by e.g. bleeding, degradation of the support matrix/tracer carrier (chemical reaction, displacement like ionic exchange, etc.) when exposed to the activating fluid (e.g. oil or water) or mechanically, for example by being washed out as a soap by the flow of the activating fluid. Thus, the amount of released tracer material will be a function of the local activating fluid flow rate or production rate. The tracers will follow the produced fluids and will be detected somewhere downstream along the production line. Information about the location, type and production rate of the concerned fluids will be a function of the amount and the type of released and measured tracers.

The unique feature of this invention is the confinement of an easy detectable tracer (any kind) in an arrangement where the tracer is released by changes in the local environment of the tracer. The changes in the environment can be changes in the flow, fluid composition, temperature, pressure or other chemical/physical condition. Such changes may even be related to the local microbiology. The tracer is placed in such a way that specific information from sections of a well is obtained when the tracer is released and detected. The tracer may be a chemical substance in itself, be produced as a chemical substance because of an event, a larger macromolecule (DNA, protein sequence etc.), particles or any other entity which can be easily detected in a water phase, an oil phase, a gas phase or a fluid mixture. The negative influence of chemicals in the reservoir formation or zones should be insignificant.

Possible detection methods may be based on optical techniques, acoustic, magnetic, capacitance, PCR, and microwaves methods, etc. The proper method depends on the applied tracers and how/where they are released.

### EXAMPLES

The examples/cases given are only intended to illustrate some of the possible applications of "intelligent chemicals and microbiology" in wells in a reservoir. These examples must not be used to limit the scope of the inventive idea.

Neither the method for placement of different tracers, the location in the reservoir, nor the distance from tracers to the production well are critical for the method described and must not be regarded as a limit of this invention. Typical methods for placement of tracers in the production well that will be used in the present invention are well known squeeze methods or directly placement in the "sand pack" during the completion of the well.

The tracers can be synthetic and/or natural chemicals, monomeric compounds or polymers which can be detected and give information about their origin (specific zone/section), different flow rates and environmental data (e.g. oil, water, gas, water breakthrough, local variations in pH, salinity, hydrocarbon composition, temperature, pressure, microorganisms, etc.) of the different zones/sections in the reservoir at all times.

The tracers may be detected by different techniques such as optical fibers, spectrophotometric methods, PCR techniques combined with sequential analysis, or by chromatographic methods. The invention is not restricted to the above mentioned techniques.

### 1. Examples on behavior and "delivery" of tracers.

**1.1. Tracer molecules covalently linked to polymers.**

In order to ensure sufficient attachment of tracers (high concentrations) to the formation, it is preferred to use tracers linked covalently or by ionic interactions to polymers which are specially designed to adhere to the formation. A high degree of functionality, e.g. carboxylic acids, sulfonic acids, phosphonic acids, phosphates and hydroxy groups and co-operative effects, e.g. ionic and hydrogen binding may be introduced in the polymers due to multiple binding sites that enhances binding to the formation. By varying the bond between the tracer and the polymer, release mechanism and rate can be controlled. The mechanisms for release of tracers may be as follows:
- Release of tracers from the polymer by breaking the "binding" between these moieties regardless of type of binding (hydrophobic, ionic or chemically covalent).
- Release of tracer by degradation of the polymer resulting in small oligomers containing tracers (solved) in the production stream. The characteristics of the oligomers/polymers will influence the solubility of the mentioned fragments, including the tracer, in different media.
- A combination of the two methods mentioned above.

### 1.2. Tracer molecules covalently linked to the surface of polymer particles (poly or monosized particles)/inorganic particles or tracers immobilized/encapsulated in such particles.

It is also possible to bind tracers to different organic particles (carriers). The "carriers" are placed in different zones/sections, and their size, chemical composition and functionality must not prohibit free flow of oil/fluids within the reservoir. Some examples of tracer carrier systems are as follows:
- Tracers attached to polymers in a network (gel) which are placed in the reservoir formation or in the "sand pack". The polymer system should not block the pores in the formation. Release of the tracers is achieved by breaking the binding between the polymer and tracer and/or by disintegration of the gel itself.
- Tracers can be attached to the polymer chains building up the polymer particles, immobilized in the polymer matrix or attached only to the surface of the particles. The particles can be placed in the reservoir formation or in the sand pack. The release of tracers is achieved by breaking the binding between the polymer particle (any carrier) and the tracer, and/or by disintegration of the particle itself in such a way that the tracers leaking out from the carrier/matrix.

Degradation of the polymer particles (or carriers) may be controlled by the use of specially defined cross-linkers. During manufacturing of the polymer particles (including the introduction of tracers) the cross-linker is stable, but in special environments the cross-linker degrades or swells, and the polymer matrix disintegrates into soluble polymers. The cross-linkers can be modified to be sensitive towards e.g. pH, temperature, water (hydrolysis) or special chemicals added to the zones/sections. It is also possible to use wax systems where the tracers are immobilized in the wax (tablets) and the tracers are released when the oil (oil soluble wax) or water (water soluble wax) comes in contact with the wax tablets.

### 2. Release of tracer from water-active matrix.

The production of water from a specific zone in the reservoir formation may be monitored by the invention. One way to do this would be to make a polymeric matrix or particle with a chemical tracer that is released by contact with water at the given conditions, e.g. encapsulation. The chemical tracers may be perlluorinated hydrocarbons such as perfluoro buthane (PB), perfluoro methyl cyclopentane (PMCP), perfluoro methyl cyclohexane (PMCN) or perfluoroethers that can be detected by mass spectroscopy (MS) or electron capture detection (ECD). Various specific perfluorinated hydrocarbons may be used for the different zones of the formation in order to monitor the water production from these zones. Tracers may also be released by chemical reactions induced by sudden changes in the water phase like pH, ionic strength or heat. Such examples may be degradation of organic anhydrides, esters or amides. The polymers used for encapsulation of the tracers may be commercially available polymers or copolymers that will degrade in water such as poly acrylamide (PAA), poly ethylenglycols (PEG), poly lactic acid (PLA), poly glycolic acid (PGA) and copolymers thereof.

The event of a water breakthrough can be detected if the formation (or a plug located in the formation) releases a detectable tracer when the water or brine passes. One way to do this would be to attach chemically a compound that is released by contact with water at the conditions given. Such a compound may by organic anhydrides. The released component can be detected downstream.

### 3. Release of tracer from hydrocarbon-active matrix.

The production of hydrocarbons from a specific zone of the formation may be monitored by the invention. One way to do this would be to make a polymeric matrix or particle with a chemical tracer that is released by contact with hydrocarbons at the given conditions, e.g. encapsulation. Such chemical tracers may be perfluorinated hydrocarbons such as perfluoro buthane (PB), perfluoro methyl cyclopentane (PMCP), perfluoro methyl cyclohexane (PMCH) or perfluoroethers that can be detected by mass spectroscopy (MS) or electron capture detection (ECD). Various specific perfluorinated hydrocarbons may be used for the different zones/sections of the formation in order to monitor the hydrocarbon production in different zones/sections. Tracers may also be released by chemical reactions induced by sudden changes in oil phase pressure, heat or changes in solubility capability (heavy or light petroleum fractions). Particle or polymer swelling may be one of such mechanisms releasing the tracer. The polymers used for encapsulation of the tracers may be commercially available polymers or copolymers that will degrade in hydrocarbons such as poly methyl methacrylates (PMMA), poly methylcrylates, poly ethylenglycols (PEG), poly lactic acid (PLA), poly glycolic acid (PGA) and copolymers thereof.

### 4. Detection of reservoir microorganisms

The presence of microorganisms in oil reservoirs may be demonstrated in produced water by traditional isolation and cultivation techniques. Such methods are quite time-consuming. In offshore oil reservoirs sulphate reducing bacterias (SRB) are often dominating, and these microorganisms produce H₂S during growth. These organisms are highly unwanted in the produced fluid/oil, because they may cause souring of the reservoir, unwanted plugging of pores in the formation and/or results in corrosions. The activity of SRB's may be demonstrated by measuring the level of H₂S in the produced water.

Growth of microorganisms in reservoirs can be identified by online monitoring using different fluorogenic and/or chromogenic enzyme substrates. The substrates can be covalently bonded or adsorbed to a matrix, e.g. , a polymer or polymer-particles, which are placed in the reservoir. Microorganisms in the reservoir produce extracellular enzymes (biocatalysts), for example proteases/peptidases and lipases. By using appropriate substrates, these enzymes will catalyze a cleavage of the substrates and the fluorogenic/chromogenic part of the substrates (the tracer part) will be released. The fluorophores/chromophores can be detected online somewhere along the production line downstream. By using different fluorogenic/chromogenic compounds in different localization of the reservoir, information about microbial activity in the different zones/sections can be obtained.

The substrates used in the method of the present invention are generally substrates for extracellular enzymes present in almost all microorganisms, for example lipases and proteases/peptidases. Lipases hydrolyze the ester bond in fats (esters of glycerol's and fatty acids) and esters of two carboxylic acids, and proteases cleave peptide bonds between amino acids in proteins and peptides. The substrates are covalently bond to a matrix in one end and to a fluorogenic/chromogenic compound (the tracer) in the other end. The carboxyl group of the end amino acid of a peptide may for example be conjugated to an amine-containing fluorophore/chromofore (the tracer compound) to create a fluorogenic/chromogenic peptidase substrate. The substrates may be bonded to the matrix for example via an amino group in a protein/peptide substrate or a carboxyl group in a fat/triglyceride substrate. The tracer materials can be placed in the reservoir formation as described elsewhere.

### 5. Detection of tracers by optical methods

One of many possible tracers is to use fluorescent or phosphorescent tracer particles/materials/chemicals. When such particles/materials/chemicals are used as tracers, they can be detected anywhere outside the reservoir along the production line from the well head to the receiving unit on land, ship or platform using for example a fiber optical technique. The on-line detection by optical fibers has many advantages including simple, fast and reliable operation. The optical probe can be made very small and flexible so it can be placed almost anywhere in the production system. Figure 1 shows a simple schematic illustration of a horizontal well 1 and a vertical well 2 divided into different zones A, B, C and D (3). The zones A-D are equipped with various tracers with different physical and/or chemical properties such as tracer particles/chemicals with different emission wavelengths. The tracers are placed in known reservoir zones/regions. When the tracers are released, they will follow the main flow toward the receiving unit (not shown in Figure 1). The concentration and passing frequency of different tracer particles can be detected by using a proper detection technique. The detection assembly (4) can be placed along the main flow direction e.g. on the top of the well, somewhere along the transporting pipeline or even just before the receiving unit.

Local motion of a single particle/chemical in a fluid flow can be determined using optical fibers. This measuring principle can be applied for simultaneous determination of flow properties of various particle groups in a flow using different tracer particles. These tracer particles may have the same physical properties apart from the radiation properties. This can be achieved e.g. by a fluorescent dye impregnation of the tracer particles. It is then possible to distinguish between the radiation from various tracer particles.

Figure 9 is a schematic illustration of a method for selection of desired emission spectra for detection of individual tracer particles with different spectra using optical filters. (I) shows whole spectra of the reflected and emitted lights from the carrying fluid and tracer particles A-D, together with the emission spectrum for the laser. The different tracer particles A-D may initially be arranged in the well as illustrated in Figure 1. The reflected light from the light source (laser) and the fluorescent light from the various tracer particles A-D may be separated using proper optical filter combinations, such that only the light from a certain tracer particle group is converted to electric signals. In Figure 9 optical bandpass filters block the unwanted light spectra and transmit the desired light spectra as illustrated in (II)-(V). The electric signals from each detector are amplified and converted separately and finally recorded on a file or displayed on the monitor of a computer. The choice of fluorescent tracer particles or chemicals and optical filter combination is based on light spectral analyses. Using this technique, the obtained signals from passage of each tracer particle group is similar to those from a single particle group and can be treated the same. By this technique it is possible to monitor the reservoir dynamics and detect all tracer groups from various well sections simultaneously.

### 6. Experiments with oil reservoir simulation system (not according to invention)

A simple column filled with glass beads (2-3 mm) was set up. The column had the dimensions as follows, length 11.5 cm, diameter 5.5 cm and a total volume of 273 cm³. A glass sinter in the bottom of the column acted as a bed for the beads. A peristaltic pump was coupled to the lower end of the column, and a drain was placed at the top. The arrangement made it possible to fill the column from the bottom with no air bubbles trapped.

The pump could run in both directions, thus enabling flows from both the bottom and up and the contrary from the top and down. The density of the liquids determines the direction of the flow. For water to replace oil the flow is from bottom and up, but for oil to replace the water the flow is from top and downwards, both to ensure a plug flow.

An initial goal with this set-up was to demonstrate the possibility to detect a change from one phase to the other. When the column is filled with oil, it is important to detect that the water coming out have been passing through a certain volume or part of the column. A water soluble, but not oil soluble, tracer was then placed in a zone in the bulk of the beads. This tracer was strongly coloured making a visual detection easy.

### Detection of water passing through tracer experiment above

Initially the column was filled with lamp oil and there was no colour whatsoever in the liquid. Clear liquid was coming from the top of the column. The inflow at the bottom changed to water and water replaced the oil from beneath. This is typically a plug flow. When the water hit the zone in the bead packing where the coloured tracer was placed (Methylthymol Blue, water soluble, typically a few milligrams), the water phase changed its colour from transparent to blue. The experiment demonstrated that the water phase passed through a predefined zone in the column and that this could be monitored.

The visual detection of a coloured dye is not a very sensible technique and more sensible techniques were tested. By using a fluorescent dye and using ultra violet light to trigger the fluorescence, a far more sensible system was achieved. Not only was visual detection by the eye possible, but also the use of a spectrometer in conjunction with an ultra violet source. The detection limits reachable with this technique is low enough to utilize this method in simulated oil well structures. The experiment was performed by placing a small amount (typically a few milligrams) of Fluorescein in the glass bead packing. A UV lamp at 265 nm illuminated the column and the fluorescent dye started to emit visible light when exposed to the UV radiation. In this experiment no instrumental detection was needed since the fluorescent light emitted by the dye was strong enough to be visible to the naked eye. This experiment demonstrated the principle that water flowing through a predefined zone in the column can be detected and monitored. It also demonstrated that a fluorescent dye is a very powerful technique for this kind of monitoring.

### Detection of oil passing through the tracer experiment above

Initially the column was filled with water and there was no colour in the water. Clear water was coming from the top of the column. The flow was then reversed and oil drawn from the top and down the column by means of a reversed flow of the peristaltic pump. The oil replaced the water from above, thus yielding a plug flow. In a predefined zone in the column the oil phase hits the tracer, in this case a dye that was only soluble in oil and not in water (Oil Blue N, typically a few milligrams). The oil that had passed through this zone was strongly coloured and could easily be detected visually. This experiment demonstrated that an oil phase that has passed through a predefined zone in the column can be detected, in this case with a coloured dye.

### Sensitivity of tracers.

In these simple experiments only dye tracers were used. Achieving the sensitivity needed for real experiments normally other tracers must be used. Fluorocarbon compounds yield an extremely high sensitivity in the electron capture detector (ECD), and a combination of a suitable fluorocarbon and GC/ECD method is a known and suitable technique for this purpose. The use of fluorocarbons in oil well monitoring is a well-established method in the oil industry.

### Environmental influence of PerFluoroCarbons.

The perfluorocarbons has a large global warming potential (GWP). The environmental impact of these compounds will cause these kinds of molecules to be phased out. It is therefore of interest to look for tracers that is environmentally acceptable, e.g. has no ozone depletion potential and a very low or zero GWP. This must be combined with the other wanted properties of the tracers, like good chemical stability and a very high sensitivity with respect to the electron capture detector (ECD) and negative ion chemical ionization mass spectrometry, NCl-MS.

The new type of compound developed as replacements for perfluorocarbons, the Perfluoroethers (e.g. CHF2-O-CF2-O-CHF2) that is a very good and environmentally acceptable alternative to the fluorocarbons. These compounds exist with very different chain lengths thereby yielding a lot of different trace molecules to be used. These ethers are also slightly water soluble making them easy to detect in the water phase.

### Dye in water soluble matrix

An quantity of 20g Polyvinylpyrrolidine K90 was dissolved in 80g of water with magnetic stirring close to the boiling point. A few mg of water soluble methylthymol blue was added, and the solution was brought to room temperature. The brownish/yellow transparent and viscous liquid was kept at 50°C for 48 h. The resulting solid polymer was broken into pieces and used in the experiments. A piece of the material was placed in water, and another one in transparent lamp oil. The piece in the water phase dissolved, giving a blue transparent solution. Nothing happened in the oil phase. The tracer containing polyvinylpyrrolidine matrix was placed in the elluation column as described above, and elluted from below with lamp oil. The tracer was stable under these conditions. The elluent was then exchanged to water. The tracer containing matrix began to dissolve when the water reached this level in the column, and the matrix started to dissolve releasing the tracer color. The release of the tracer was much slower compared to the pure dye above.

Having described preferred embodiments of the invention it will be apparent to those skilled in the art that other embodiments incorporating the concepts may be used. These and other examples of the invention illustrated above are intended by way of example only and the actual scope of the invention is to be determined from the following claims.

## Claims

1. A method for monitoring hydrocarbon and water production from a hydrocarbon reservoir (2) or injection well and detection of phenomena including local variations in pH, salinity, hydrocarbon composition, temperature, pressure, microorganisms, and a difference between production of formation and injection water, the method comprising the steps of:
dividing regions around wells in the reservoir into a number of zones, and **characterised by** the steps of,
chemically immobilizing specific chemically intelligent tracers with unique characteristics for each zone into a well completion, a filter, a casing or construction surrounding the well in each zone, releasing the tracers as a function of a specific event, wherein said tracers are immobilized by means of at least one polymer capable of adhering to the formation, and wherein the tracers being covalently bonded or bonded by ionic interactions to said polymer, and
detecting the tracers downstream to monitor said zones.

2. A method according to claim 1, wherein the tracers are released at contact with either an oil, gas or water flow.

3. A method according to claim 2, further comprising the step of:
using as tracers various perfluorinated hydrocarbons encapsulated in a polymer matrix, the matrix being sensitive to water and hydrocarbon, respectively

4. A method according to claim 1, further comprising the step of:
releasing the tracers from the polymer by breaking the binding between the polymer and the tracer

5. A method according to claim 1, further comprising the step of
releasing the tracers by degradation of the polymer, resulting in small oligomers or chemicals containing tracers in the production stream.

6. A method according to claim 1, further comprising the steps of:
controlling the degradation of the polymer by using specially defined cross-linkers, the cross-linkers being modified to be sensitive towards specific events like e g. pH, temperature, water or special chemicals added to the zones.

7. A method according to claim 1, wherein the tracer is an oligonucleotide with special functional groups.

8. A method according to claim 1, further comprising the step of:
injecting specific triggers into the formation through an injection system triggering the release of tracers.

9. A method according to claim 1, further comprising the step of:
performing short time and long time monitoring of the various zones in the hydrocarbon reservoir by using a first group of tracers and a second group of tracers, the tracers being released over a short time , and gradually over a long time, respectively.

10. A method according to claim 1, further comprising the step of:
detecting the presence of microorganisms in the different zones by using different fluorogenic or chromogenic enzyme substrates as tracers, the substrates and the fluorogenic/chromogenic part being cleaved by extracellular enzymes produced by the microorganisms in the reservoir.

11. A method according to claim 1, using fluorescent, phosphorescent, magnetic particles or fluids, coloured particles, DNA or microorganisms as tracers.

12. A method according to claim 1, further comprising the step of
detecting the tracers by optical, spectroscopic, chromatographic, acoustic, magnetic, capacitive, PCR or microwave techniques, or by any combination of these techniques.

## Patentansprüche

1. Verfahren zur Überwachung der Produktion von Kohlenwasserstoff und Wasser aus einem Kohlenwasserstoff-Reservoir (2) oder einem Injektionsbrunnen und zur Erfassung von Phänomenen, die lokale Veränderungen des pH-Werts, des Salzgehalts, der Kohlenwasserstoffzusammensetzung, der Temperatur, des Drucks, der Mikroorganismen sowie einen Unterschied zwischen der Produktion von Formationswasser und Injektionswasser umfassen, wobei das Verfahren die folgenden Schritte umfasst:
- Aufteilen von Bereichen, die sich in der Nähe von Brunnen im Reservoir befinden, in eine Anzahl von Zonen, und **gekennzeichnet durch** die folgenden Schritte:
- chemisches Immobilisieren spezifischer, chemisch intelligenter Tracer mit für jeden Bereich eindeutigen Merkmalen in eine Bohrlochkomplettierung, einen Filter, ein Gehäuse oder einen Aufbau, der den Brunnen umgibt, in jedem Bereich, Freisetzen der Tracer als Funktion eines spezifischen Ereignisses, wobei die Tracer mit Hilfe mindestens eines Polymers immobilisiert werden, das an der Formation anhaften kann, und wobei die Tracer kovalent oder **durch** ionische Wechselwirkungen an das Polymer gebunden sind, und
- nachgeordnetes Erfassen der Tracer, um die Zonen zu überwachen.

2. Verfahren nach Anspruch 1, wobei die Tracer bei Kontakt mit entweder einem Öl, einem Gas oder einem Wasserstrom freigesetzt werden.

3. Verfahren nach Anspruch 2, weiterhin umfassend den folgenden Schritt:
Verwenden verschiedener perfluorierter Kohlenwasserstoffe als Tracer, die in einer Polymermatrix eingekapselt sind, wobei die Matrix gegen Wasser beziehungsweise Kohlenwasserstoff empfindlich ist.

4. Verfahren nach Anspruch 1, weiterhin umfassend den folgenden Schritt:
Freisetzen der Tracer von dem Polymer durch Aufbrechen der Bindung zwischen dem Polymer und dem Tracer.

5. Verfahren nach Anspruch 1, weiterhin umfassend den folgenden Schritt:
Freisetzen der Tracer durch Abbau des Polymers, was zu kleinen Oligomeren oder Chemikalien führt, die Tracer im Produktionsstrom enthalten.

6. Verfahren nach Anspruch 1, weiterhin umfassend folgende Schritte:
Steuern des Abbaus des Polymers unter Verwendung speziell definierter Vemetzer, wobei die Vernetzer so modifiziert sind, dass sie gegen spezifische Ereignisse wie z.B. pH-Wert, Temperatur, Wasser oder spezielle Chemikalien, die den Zonen zugegeben werden, empfindlich sind.

7. Verfahren nach Anspruch 1, wobei der Tracer ein Oligonukleotid mit speziellen funktionellen Gruppen ist.

8. Verfahren nach Anspruch 1, weiterhin umfassend den folgenden Schritt:
Injizieren spezifischer Trigger in die Formation über ein Injektionssystem, die die Freisetzung von Tracern triggern.

9. Verfahren nach Anspruch 1, weiterhin umfassend den folgenden Schritt:
Durchführen einer Kurzzeit- und Langzeitüberwachung der verschiedenen Zonen in dem Kohlenwasserstoff-Reservoir unter Verwendung einer ersten Gruppe von Tracern und einer zweiten Gruppe von Tracern, wobei die Tracer über einen kurzen Zeitraum beziehungsweise nach und nach über einen langen Zeitraum hinweg freigesetzt werden.

10. Verfahren nach Anspruch 1, weiterhin umfassend den folgenden Schritt:
Erfassen des Vorhandenseins von Mikroorganismen in den verschiedenen Zonen unter Verwendung unterschiedlicher fluorogener oder chromogener Enzymsubstrate als Tracer, wobei die Substrate und der fluorogene /chromogene Teil durch extrazelluläre Enzyme gespalten werden, die von den Mikroorganismen in dem Reservoir gebildet werden.

11. Verfahren nach Anspruch 1, das fluoreszente, phosphoreszente, magnetische Partikel oder Fluide, farbige Partikel, DNA oder Mikroorganismen als Tracer verwendet.

12. Verfahren nach Anspruch 1, weiterhin umfassend den folgenden Schritt:
Erfassen der Tracer durch optische, spektroskopische, chromatographische, akustische, magnetische, kapazitive, PCR- oder Mikrowellenverfahren oder durch jede beliebige Kombination dieser Verfahren.

## Revendications

1. Procédé de contrôle de la production d'hydrocarbure et d'eau, à partir d'un réservoir d'hydrocarbure (2) ou d'un sondage d'injection, et de détection de phénomènes incluant des variations locales de pH, salinité, composition des hydrocarbures, température, pression, microorganismes, et une différence entre la production d'eau de formation et d'eau d'injection, le procédé comprenant les étapes consistant à :
diviser des régions autour des puits dans le réservoir en un certain nombre de zones, et **caractérisé par** les étapes consistant à :
immobiliser chimiquement des traceurs spécifiques chimiquement intelligents avec des caractéristiques uniques pour chaque zone dans une complétion de puits, un filtre, un tubage ou une construction entourant le puits dans chaque zone, libérer les traceurs en fonction d'un événement spécifique, où lesdits traceurs sont immobilisés à l'aide d'au moins un polymère capable d'adhérer à la formation et où les traceurs sont liés de manière covalente ou liés par interactions ioniques audit polymère, et
détecter les traceurs en aval pour contrôler lesdites zones.

2. Procédé selon la revendication 1, dans lequel les traceurs sont libérés au contact d'une huile, de gaz ou d'eau.

3. Procédé selon la revendication 2, comprenant en outre, l'étape consistant à :
utiliser comme traceurs, différents hydrocarbures perfluorés, encapsulés dans une matrice polymère, la matrice étant sensible à l'eau et aux hydrocarbures, respectivement.

4. Procédé selon la revendication 1, comprenant en outre, l'étape consistant à :
libérer les traceurs du polymère par cassure de la liaison entre le polymère et le traceur.

5. Procédé selon la revendication 1, comprenant en outre, l' étape consistant à :
libération des traceurs par dégradation du polymère, ce qui résulte en de petits oligomères ou composés chimiques contenant les traceurs dans le courant de production.

6. Procédé selon la revendication 1, comprenant en outre, l'étape consistant à :
contrôler la dégradation du polymère en utilisant des agents de réticulation spécifiquement définis, les agents de réticulation étant modifiés pour être sensibles à des événements spécifiques comme par exemple, le pH, la température, l'eau ou des composés chimiques spéciaux ajoutés aux zones.

7. Procédé selon la revendication 1, où le traceur est un oligonucléotide avec des groupes fonctionnels spéciaux.

8. Procédé selon la revendication 1, comprenant en outre, l'étape consistant à :
injecter des déclencheurs spécifiques dans la formation par un système d'injection déclenchant la libération des traceurs.

9. Procédé selon la revendication 1, comprenant en outre, l'étape consistant à :
réaliser un contrôle à court terme et à long terme des différentes zones dans le réservoir d'hydrocarbure en utilisant un premier groupe de traceurs et un deuxième groupe de traceurs, les traceurs étant libérés en peu de temps, et graduellement sur une période longue, respectivement.

10. Procédé selon la revendication 1, comprenant en outre, l'étape consistant à :
détecter la présence des microorganismes dans les différentes zones en utilisant différents substrats enzymatiques fluorogènes ou chromogènes comme traceurs, les substrats et la partie fluorogène/chromogène étant clivés par les enzymes extracellulaires produites par les microorganismes dans le réservoir.

11. Procédé selon la revendication 1, utilisant des particules ou fluides fluorescents, phosphorescents, magnétiques, des particules colorées, l'ADN ou des microorganismes comme traceurs.

12. Procédé selon la revendication 1, comprenant en outre, l' étape consistant à :
détecter les traceurs par des techniques optiques, spectroscopiques, chromatographiques, acoustiques, magnétiques, capacitives, de PCR ou par micro-ondes, ou par une combinaison de ces techniques.
